# EUROPÄISCHE PATENTANMELDUNG

(11) **EP 0 705 819 A1**
(43) Veröffentlichungstag der Anmeldung: **10.04.1996**
(21) Anmeldenummer: 95112766.1
(22) Anmeldetag: 14.08.1995
(51) Int. Cl.: C07D 211/90, A61K 31/44

(54) **1,4-Dihydropyridin-3,5-dicarbonsäureestern und ihre Verwendung als selektive Kaliumkanalmodulatoren**

(30) Priorität: 25.08.1994 DE 4430094
(71) Anmelder: BAYER AG, D-51368 Leverkusen (DE)
(72) Erfinder: Urbahns, Klaus, Dr., D-45115 Wuppertal (DE); Behner, Otto, Dr., D-42113 Wuppertal (DE); Goldmann, Siegfried, Dr., D-42327 Wuppertal (DE); Heine, Hans-Georg, Dr., D-47800 Krefeld (DE); Junge, Bodo, Dr., D-42399 Wuppertal (DE); Schohe-Loop, Rudolf, Dr., D-42327 Wuppertal (DE); Wehinger, Egbert, Dr., D-42115 Wuppertal (DE); Wollweber, Hartmund, Dr., D-42113 Wuppertal (DE); Sommermeyer, Henning, Dr., D-51061 Köln (DE); Wittka, Thomas, Dr., D-51069 Köln (DE); de Vry, Jean-Marie-Viktor, Dr., D-51503 Rösrath (DE); Glaser, Thomas, Dr., D-51491 Overath (DE)

(57) **Zusammenfassung**

Die vorliegende Erfindung betrifft die Verwendung von teilweise bekannten 1,4-Dihydropyridin-3,5-dicarbonsäureestern der allgemeinen Formel (I) 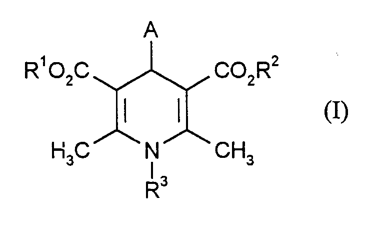
in welcher
A, R¹-R³ die in der Beschreibung angegebene Bedeutung haben, als Arzneimittel, insbesondere zur Behandlung des zentralen Nervensystems und neue ausgewählte Wirkstoffe.

## Beschreibung

Die vorliegende Erfindung betrifft die Verwendung von teilweise bekannten 1,4-Dihydropyridin-3,5-dicarbonsäureestern als Arzneimittel, insbesondere zur Behandlung des zentralen Nervensystems und neue ausgewählte Wirkstoffe.

Aus DOS 40 11 695 sind bereits 1-Alkyl- und 1-Cycloalkyl-(1,4-dihydropyridin)-3,5-dicarbonsäureester mit einer rheologischen Aktivität bekannt. Aus DOS 1 813 436 und DOS 1 923 990 sind auch 1-N-substituierte Dihydropyridine mit Coronar- und Kreislaufwirkung bekannt die Einfluß nehmen auf die Gefäße des Kreislaufs. Die cerebrale Wirkung des speziellen Dihydropyridins Nimopidin ist bereits in DOS 2 815 578 beschrieben.

Es wurde nun gefunden, daß die 1,4-Dihydropyridin-3,5-dicarbonsäureester der allgemeinen Formel (I), 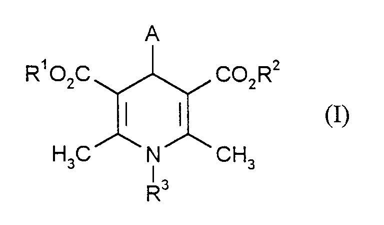
in welcher
- A: für Aryl mit 6 bis 10 Kohlenstoffatomen oder Pyridyl steht, die gegebenenfalls bis zu 3-fach gleich oder verschieden durch Nitro, Cyano, Halogen, Trifluormethyl oder durch geradkettiges oder verzweigtes Alkylthio mit bis zu 6 Kohlenstoffatomen substituiert sind,
- R¹ und R²: gleich oder verschieden sind und
für Cycloalkyl mit 3 bis 6 Kohlenstoffatomen stehen oder
für geradkettiges oder verzweigtes Alkyl mit bis zu 8 Kohlenstoffatomen stehen, das gegebenenfalls bis zu 2-fach gleich oder verschieden durch geradkettiges oder verzweigtes Alkoxy mit bis zu 4 Kohlenstoffatomen, Halogen, oder durch eine Gruppe der Formel -NR⁴R⁵ substituiert ist,
worin
- R⁴ und R⁵: gleich oder verschieden sind und
Wasserstoff, Benzyl, Phenyl oder geradkettiges oder verzweigtes Alkyl mit bis zu 4 Kohlenstoffatomen bedeuten,
- R³: für Cycloalkyl mit 3 bis 6 Kohlenstoffatomen oder
für geradkettiges oder verzweigtes Alkyl mit bis zu 6 Kohlenstoffatomen steht, das gegebenenfalls durch Hydroxy, Phenyl oder durch geradkettiges oder verzweigtes Alkoxy oder Alkoxycarbonyl mit jeweils bis zu 6 Kohlenstoffatomen substituiert ist
und deren Salze
überraschenderweise eine selektive modulierende Wirkung auf calciumabhängige Kaliumkanäle großer Leitfähigkeit besitzen und somit geeignet sind zur Verwendung bei der Behandlung des zentralen Nervensystems, insbesondere bei der Bekämpfung von neuronalen Erkrankungen und bei der Behandlung von Depressionen und Demenzen und bei Sichelzellenanämie.

Vorzugsweise betrifft die vorliegende Erfindung die Verwendung solcher Dihydropyridine der Formel (I), die bei üblicher Dosierung praktisch keine relevante calciumagonistische oder calciumantagonistische Wirkung haben, sondern spezifische modulierende Wirkungen auf Kaliumkanäle besitzen.

Besonders hervorzuheben sind Verbindungen, deren Selektivitätsquotient Q_{sel.} mindestens den Wert 10 besitzt. Dieser Selektivitätsquotient wird ermittelt als Quotient aus den Daten der Kalium-Kanal-Wirkung gemäß Test 1 und den Daten aus der Calcium-Kanal-Wirkung gemäß Test 2.

### Test 1 (Kalium-Kanal-Wirkung:

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellkulturen verwendet. Aus den Daten wird die durch Ionomycin hervorgerufene Erhöhung des ⁸⁶Rb-Effluxes über den Basalefflux berechnet und als 100 % gesetzt. Die Stimulationen in Gegenwart von Prüfsubstanzen werden dann auf diesen Wert bezogen.

### Test 2 (Calcium-Kanal-Wirkung):

Die Versuche werden mit geringen Abweichungen nach D.J. Triggle (Molecular Pharmacol. 35, 541-52 (1989) durchgeführt. Dabei wird die Bindung der Substanzen an den L-Ca-Kanal durch Verdrängung von radioaktiv markiertem ³(+)-PN-200-110 an Rattenhirnmembranhomogenaten bestimmt. Die Ergebnisse werden als Ki-Werte ausgedrückt.

Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt verwendet werden Verbindungen der allgemeinen Formel (I),
in welcher
- A: für Phenyl, Naphthyl oder Pyridyl steht, die gegebenenfalls bis zu 3-fach gleich oder verschieden durch Nitro, Cyano, Fluor, Chlor, Brom, Iod, Trifluormethyl oder durch geradkettiges oder verzweigtes Alkylthio mit bis zu 4 Kohlenstoffatomen substituiert sind,
- R¹ und R²: gleich oder verschieden sind und
für Cyclopropyl, Cyclopentyl oder Cyclohexyl stehen oder
für geradkettiges oder verzweigtes Alkyl mit bis zu 7 Kohlenstoffatomen stehen, das gegebenenfalls bis zu 2-fach gleich oder verschieden durch geradkettiges oder verzweigtes Alkoxy mit bis zu 3 Kohlenstoffatomen, Fluor, Chlor, Brom oder durch eine Gruppe der Formel - NR⁴R⁵ substituiert ist,
worin
- R⁴ und R⁵: gleich oder verschieden sind und
Wasserstoff, Benzyl, Phenyl oder geradkettiges oder verzweigtes Alkyl mit bis zu 3 Kohlenstoffatomen bedeuten,
- R³: für Cyclopropyl, Cyclopentyl oder Cyclohexyl stehen, oder
für geradkettiges oder verzweigtes Alkyl mit bis zu 5 Kohlenstoffatomen steht, das gegebenenfalls durch Hydroxy, Phenyl oder durch geradkettiges oder verzweigtes Alkoxy oder Alkoxycarbonyl mit jeweils bis zu 5 Kohlenstoffatomen substituiert ist
und deren Salze.

Besonders bevorzugt sind Verbindungen der allgemeinen Formel (I),
in welcher
- A: für Phenyl, Naphthyl oder Pyridyl steht, die gegebenenfalls bis zu 2-fach gleich oder verschieden durch Nitro, Cyano, Fluor, Chlor, Brom, Trifluormethyl oder durch Methylthio substituiert sind,
- R¹ und R²: gleich oder verschieden sind und
für Cyclopropyl, Cyclopentyl oder Cyclohexyl stehen oder
für geradkettiges oder verzweigtes Alkyl mit bis zu 6 Kohlenstoffatomen stehen, das gegebenenfalls bis zu 2-fach gleich oder verschieden durch Methoxy, Fluor, Chlor, Brom oder durch eine Gruppe der Formel - NR⁴R⁵ substituiert ist,
worin
- R⁴ und R⁵: gleich oder verschieden sind und Wasserstoff, Benzyl, Phenyl, Methyl oder Ethyl bedeuten,
- R³: für Cyclopropyl, Cyclopentyl oder Cyclohexyl stehen, oder
für geradkettiges oder verzweigtes Alkyl mit bis zu 5 Kohlenstoffatomen steht, das gegebenenfalls durch Hydroxy, Phenyl oder durch geradkettiges oder verzweigtes Alkoxy oder Alkoxycarbonyl mit jeweils bis zu 3 Kohlenstoffatomen substituiert ist
und deren Salze
bei der Bekämpfung von cerebralen Erkrankungen.

Außerdem betrifft die Erfindung neue ausgewählte Verbindungen der allgemeinen Formel (I) und deren Salze,
mit den in der folgenden Tabelle angegebenen Substituentenbedeutungen:

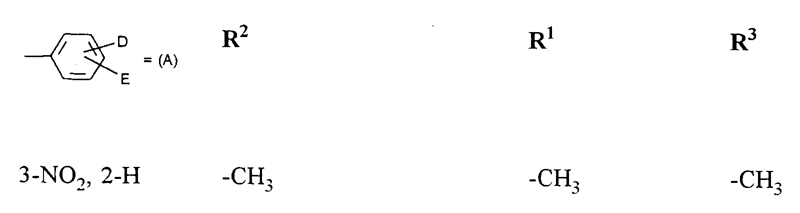

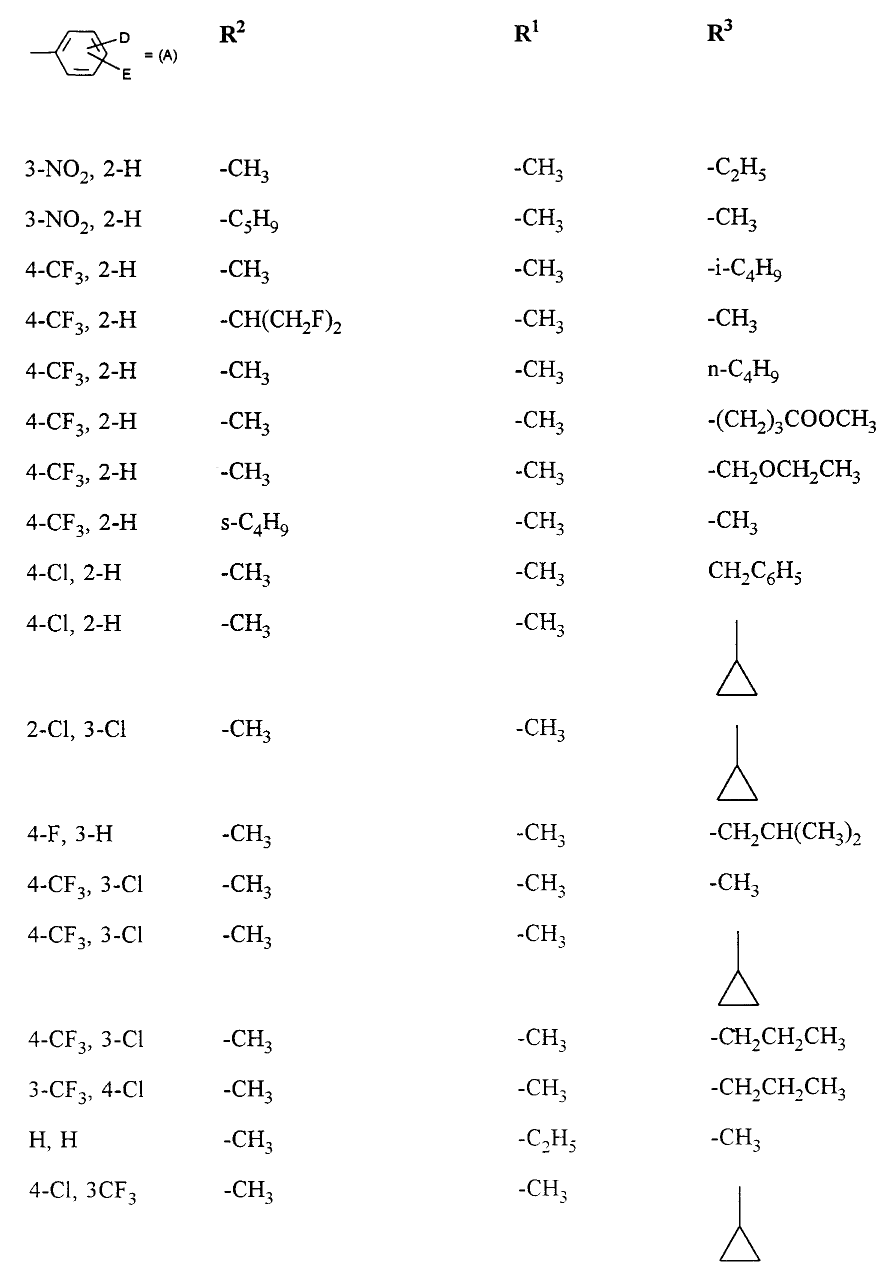

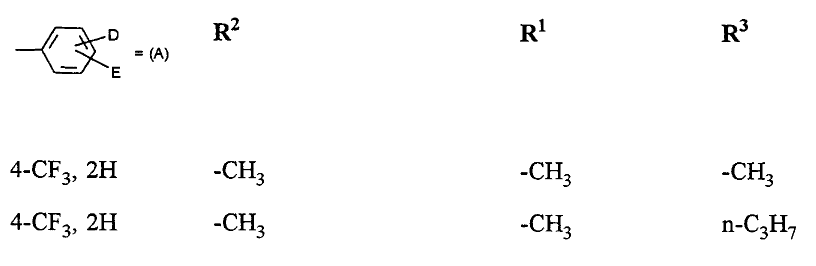

Die Verbindungen der allgemeinen Formel (I) können nach üblichen Methoden z.B. nach den in der DOS 40 11 695 beschriebenen Verfahren hergestellt werden.

Die erfindungsgemäßen bekannten und neuen Verbindungen der allgemeinen Formel (I) zeigen ein nicht vorhersehbares Wirkspektrum.

Sie sind speziell ausgewählte Kanalmodulatoren mit Selektivität für Calciumabhängige Kalium-Kanäle großer Leitfähigkeit (BK(Ca)-Kanäle), insbesondere des zentralen Nervensystems.

Aufgrund ihrer pharmakologischen Eigenschaften können sie für die Herstellung von Arzneimitteln zur Behandlung von zentral degenerativen Erkrankungen eingesetzt werden, wie z.B. bei Auftreten von Demenzen (Multiinfarktdemenz (MID), primär degenerativer Demenz (PDD), prä- und seniler Alzheimerscher Krankheit, HIV-Demenz und andere Demenzformen), Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Herstellungsbeispiele

Die neuen Verbindungen der Ausführungsbeispiele 1 bis 18 wurden in Analogie zu den Methoden aus der DOS 40 11 695 hergestellt.

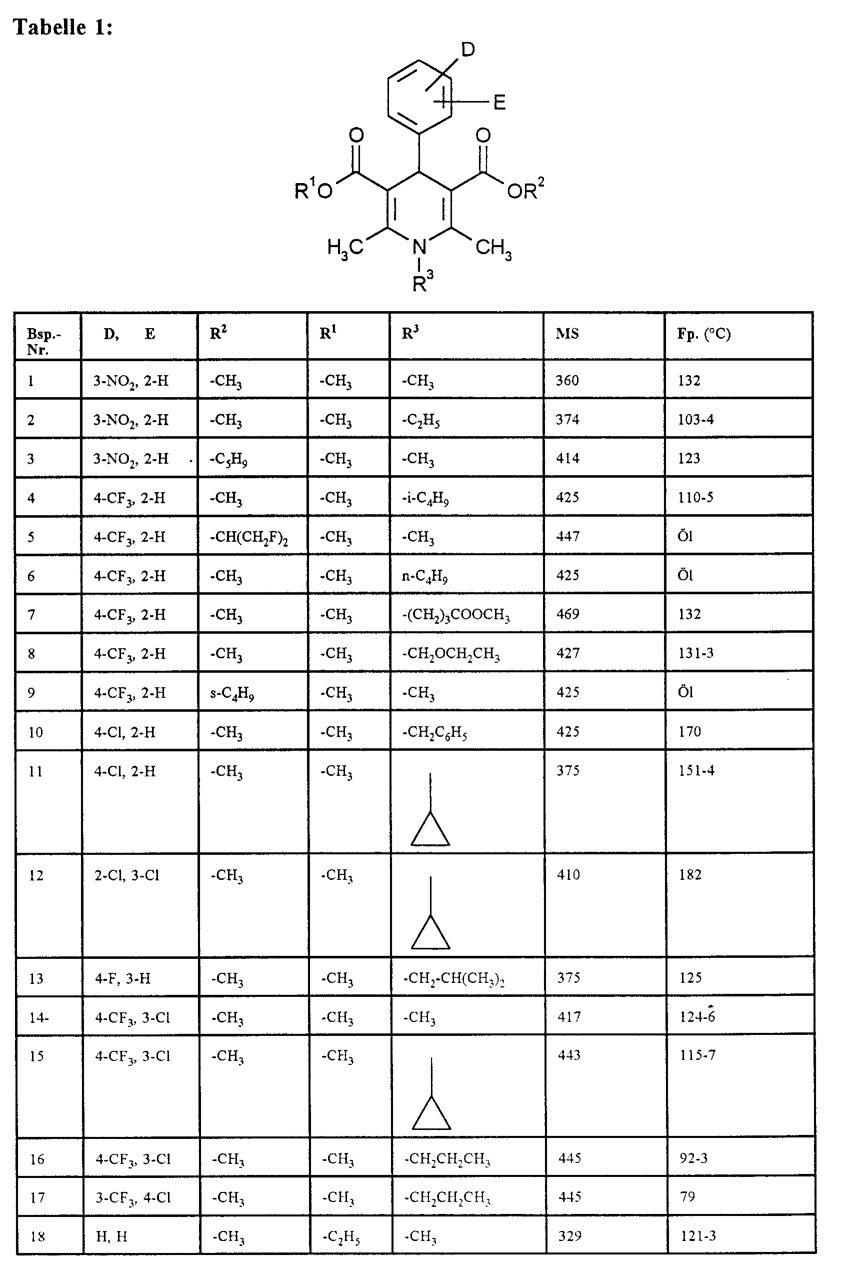

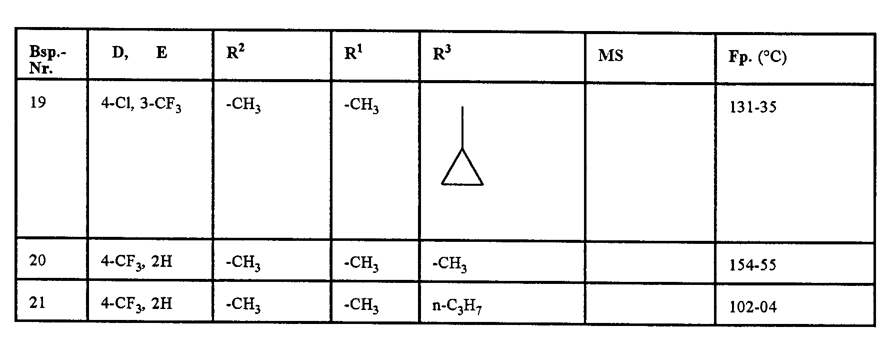

## Patentansprüche

1. Verwendung von 1,4-Dihydropyridin-3,5-dicarbonsäureestern der allgemeinen Formel (I) 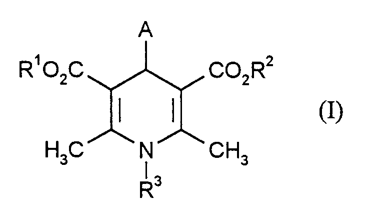 in welcher
A für Aryl mit 6 bis 10 Kohlenstoffatomen oder Pyridyl steht, die gegebenenfalls bis zu 3-fach gleich oder verschieden durch Nitro, Cyano, Halogen, Trifluormethyl oder durch geradkettiges oder verzweigtes Alkylthio mit bis zu 6 Kohlenstoffatomen substituiert sind,
R¹ und R² gleich oder verschieden sind und
für Cycloalkyl mit 3 bis 6 Kohlenstoffatomen stehen oder
für geradkettiges oder verzweigtes Alkyl mit bis zu 8 Kohlenstoffatomen stehen, das gegebenenfalls bis zu 2-fach gleich oder verschieden durch geradkettiges oder verzweigtes Alkoxy mit bis zu 4 Kohlenstoffatomen, Halogen, oder durch eine Gruppe der Formel -NR⁴R⁵ substituiert ist,
worin
R⁴ und R⁵ gleich oder verschieden sind und Wasserstoff, Benzyl, Phenyl oder geradkettiges oder verzweigtes Alkyl mit bis zu 4 Kohlenstoffatomen bedeuten,
R³ für Cycloalkyl mit 3 bis 6 Kohlenstoffatomen oder
für geradkettiges oder verzweigtes Alkyl mit bis zu 6 Kohlenstoffatomen steht, das gegebenenfalls durch Hydroxy, Phenyl oder durch geradkettiges oder verzweigtes Alkoxy oder Alkoxycarbonyl mit jeweils bis zu 6 Kohlenstoffatomen substituiert ist
und deren Salze
als Arzneimittel mit selektiver modulierender Wirkung auf Calcium-abhängige Kalium-Kanäle mit großer Leitfähigkeit.

2. Verwendung von Verbindungen der allgemeinen Formel (I) gemäß Anspruch 1 bei der Herstellung von Arzneimitteln zur Behandlung des zentralen Nervensystems.

3. Verwendung solcher Verbindungen der allgemeinen Formel (I) gemäß Anspruch 1 zur Behandlung des zentralen Nervensystems die einen Selektivitätsquotienten Qₛₑₗ von mindestens 10 besitzen.

4. Neue Verbindungen der allgemeinen Formel (I) gemäß Anspruch 1 ausgewählt aus den Ausführungsbeispielen 1 bis 18.

5. Arzneimittel mit selektiver Kalium-Kanal-modulierender Wirkung enthaltend mindestens 1 Dihydropyridin der allgemeinen Formel (I) gemäß Anspruch 1.

6. Verfahren zur Herstellung von Arzneimitteln mit selektiver Kalium-Kanalmodulierender Wirkung zur Behandlung des zentralen Nervensystems, dadurch gekennzeichnet, daß man mindestens eine Verbindung der allgemeinen Formel (I) gemäß Anspruch 1 gegebenenfalls mit üblichen Hilfsstoffen in eine geeignete Applikationsform überführt.
